# EUROPEAN PATENT SPECIFICATION

(11) **EP 1 880 722 B1**
(45) Date of publication and mention of the grant of the patent: **17.03.2010**
(21) Application number: 06015012.5
(22) Date of filing: 19.07.2006
(51) Int. Cl.: A61K 31/496, A61K 9/20, C07D 215/56

(54) **Pharmaceutical compositions of ciprofloxacin**
Pharmazeutische Zubereitungen von Ciprofloxacin
Préparations pharmaceutiques des Ciprofloxacin

(43) Date of publication of application: 23.01.2008
(73) Proprietor: Tabuk Pharmaceutical Manufacturing Co., 11844 Amman (JO)
(72) Inventor: Qassim, Sami M.A., 13110 Jordan (JO)
(74) Representative: Winkler, Andreas Fritz Ernst

(56) References cited:
- WO-A-01/12162
- WO-A-01/64183
- WO-A-02/00219
- US-B1- 6 960 356

## Description

The present invention relates in general to the field of pharmaceutical compositions, namely to a pharmaceutical composition in the form of a tablet comprising the active ingredient Ciprofloxacin. More precisely the present invention relates to a coated solid monolayer oral dosage form comprising Ciprofloxacin or a pharmaceutically acceptable salt thereof or base thereof.

### FIELD OF THE INVENTION

Ciprofloxacin is a broad spectrum fluoroquinolone antibacterial agent. Since its introduction in the 1980s, most Gram-negative bacteria have remained highly susceptible to this agent in vitro; Gram-positive bacteria are generally susceptible or moderately susceptible. Ciprofloxacin attains therapeutic concentrations in most tissues and body fluids. The results of clinical trials with Ciprofloxacin have confirmed its clinical efficacy and low potential for adverse effects. Ciprofloxacin is effective in the treatment of a wide variety of infections, particularly those caused by Gram-negative pathogens. These include complicated urinary tract infections, sexually transmitted diseases (gonorrhoea and chancroid), skin and bone infections, gastrointestinal infections caused by multiresistant organisms, lower respiratory tract infections (including those in patients with cystic fibrosis), febrile neutropenia (combined with an agent which possesses good activity against Gram-positive bacteria), intraabdominal infections (combined with an antianaerobic agent) and malignant external otitis.

Ciprofloxacin is one of the few broad spectrum antibacterials available in both intravenous and oral formulations. In this respect, it offers the potential for cost savings with sequential intravenous and oral therapy in appropriately selected patients and may allow early discharge from hospital in some instances. In conclusion, ciprofloxacin has retained its excellent activity against most Gram-negative bacteria, and fulfilled its potential as an important antibacterial drug in the treatment of a wide range of infections.

For oral therapy Ciprofloxacin tablets are available in the form of immediate release tablets of 100, 250, 500, 750 mg strengths that are typically administrated twice daily. However, new formulations have been developed to provide once-a-day Ciprofloxacin compositions to increase the compliance of a patient.

One example is Cipro® XR, an extended-release tablet provided by the company Bayer, for the treatment of complicated urinary tract infections (cUTIs) and acute uncomplicated pyelonephritis (AUP), or kidney infection. Cipro® XR is formulated to be taken just once daily to kill certain bacteria causing urinary tract infections. This formulation uses a bilayer matrix of the active ingredient ciprofloxacin, which enables two different release mechanisms. The first is a rapid release of Ciprofloxacin, which quickly distributes to the serum and tissues. This is followed by a second extended release of the active ingredient to achieve high concentrations of Ciprofloxacin in the urine for the full 24-hour dosing interval. This once-a-day formulation contributes to the compliance among patients, and thereby helps to reduce the risk of UTI relapse and subsequent resistant infection.

Cipro® XR is available in unit doses of 500 and 1000 mg. For instance, a Cipro® XR 1 g tablet contains an equivalent to 574.9 mg Ciprofloxacin base as Ciprofloxacin hydrochloride and 425.2 mg Ciprofloxacin base. This tablet is formulated as a bilayer tablet, consisting of an immediate release-layer and an erosion-matrix type controlled release layer. This tablet further contains to the active ingredients following excipients: hypromellose, crosslinked polyvinyl pyrollidine, magnesium stearate, silica colloidal anhydrous, polyethylene glycol, succinic acid and titanium dioxide.

Further US patents No. 6,261,601 and 6,960,356 (for Ranbaxy Laboratories), disclose a formulation of extended release Ciprofloxacin tablets. These pharmaceutical compositions use sodium alginate and sodium bicarbonate to produce a single layer tablet that reacts with stomach media upon ingestion and evolves gas, which is trapped in the outer layer of the hydrated alginate that add to the sustained action of alginate and keep the tablet floating in the stomach. These tablets contain 1000 mg Ciprofloxacin base as an active ingredient and the following excipients: sodium alginate, Xanthan gum, sodium bicarbonate, crosslinked polyvinyl pyrollidine, magnesium stearate and talc.

US Patent No. 6,488,962 discloses a pharmaceutical composition comprising Ciprofloxacin, wherein the retention of such tablet in the stomach is improved by using swellable dosage forms that are shaped in a manner that will prevent them from inadvertently passing through the pylorus as a result of being in a particular orientation. The planar projection of the shape is one that has two orthogonal axes of different lengths, the longer being short enough to permit easy swallowing prior to swelling while the shorter is long enough within one-half hour of swelling to prevent passage through the pylorus. This extended release tablet uses only Ciprofloxacin hydrochloride in an unit dose of 500 mg. The formula depends on the formulation of an erosion matrix that results in gradual release of the drug over 6 hours, depending on polyethylene oxide as the matrix former. This formula further contains in addition to Ciprofloxacin hydrochloride, the following additives: povidone, polyethylene oxide, magnesium stearate and Opadry® blue.

The pharmaceutical compositions mentioned above show an extended release of the active ingredient Ciprofloxacin as a result of a complex formulation. Therefore, it would be an advantage to provide a pharmaceutical composition easily to be manufactured but at the same time having similar bioavailability characteristics than the bilayer matrix formulation Cipro^{®} XR. Thus, there is a need in the prior art for a conventional tablet in form of a single layer having a fast release pattern, but at the same time having similar in-vivo data as the commonly known bilayer matrix formulation Cipro® XR.

### SUMMARY OF THE INVENTION

The present invention provides a novel pharmaceutical composition in the form of a tablet, more precisely the present invention provides a single layer formulation in the form of a coated solid oral dosage form as defined in claim 1.

### BRIEF DESCRIPTION OF THE DRAWINGS

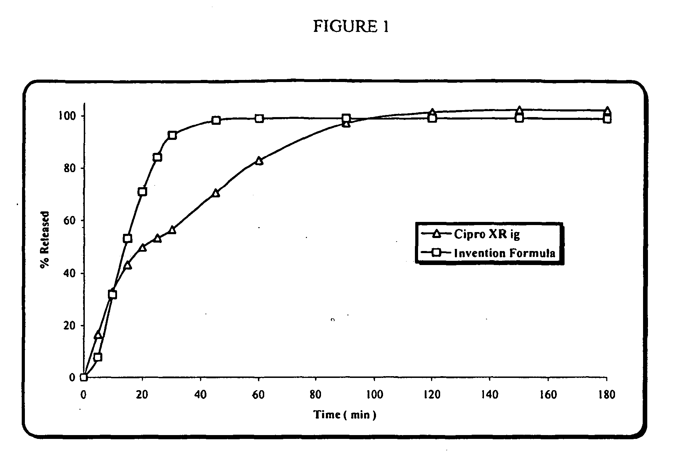

Figure 1: shows the release rate of Ciprofloxacin from the coated solid oral dosage form of the present invention (Quinox® XR) in an unit dose of 1 g Ciprofloxacin and of the known Cipro® XR 1 mg composition provided by the company Bayer.
Figure 2: shows the mean Ciprofloxacine plasma concentration - time profile after administration of the coated solid oral dosage form of the present invention (Quinox® XR) in an unit dose of 1 g Ciprofloxacin and of the known Cipro® XR 1 g composition provided by the company Bayer.

### DESCRIPTION OF THE PREFERRED EMBODIMENTS OF THE INVENTION

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

Unless defined otherwise, all technical and scientific terms used herein have the same meanings as commonly understood by one of ordinary skill in the art to which this invention belongs. Although any methods and materials similar or equivalent to those described herein can also be used in the practice or testing of the present invention, the preferred methods and materials are now described.

As outlined above there is a need in the prior art to provide a single layer pharmaceutical composition comprising Ciprofloxacin or a pharmaceutically acceptable salt or base thereof having similar bioavailability characteristics than the corresponding Cipro® XR.

Herein under, all weight percentages are based on the total weight of coated solid oral dosage forms.

Thus, in one aspect the present invention provides a coated solid oral dosage form as defined in claim 1.

In one embodiment of the coated solid oral dosage form according to the present invention the active agent is present in an amount of 70 % to 80 % by weight. Preferably the active agent is present in an amount of 75 % to 78 % by weight, and more preferably in an amount of 76 % to 77 % by weight.

In a preferred embodiment the active agent is present in an unit dose of 500 mg or 1000 mg. Preferably the active agent is present in an unit dose of 1000 mg.

In a further preferred embodiment the active agent Ciprofloxacin hydrochloride is present in an amount of 40 % to 50 % by weight. Preferably Ciprofloxacin hydrochloride is present in an amount of 45 % to 50 % by weight, more preferably in an amount of 45 % to 48 % by weight, even more preferably in an amount of 46 % to 47 % by weight.

In another preferred embodiment the Ciprofloxacin base is present in an amount of 25 % to 35 % by weight. Preferably Ciprofloxacin base is present in an amount of 25 % to 31 % by weight, more preferably in an amount of 28 % to 31 % by weight, even more preferably in an amount of 29 % to 30 % by weight.

In the dosage form of the invention the binder is selected from the group consisting of starches, microcrystalline cellulose, Povidone K30, hydroxypropyl cellulose, hydroxyl ethyl cellulose, hydroxypropylmethyl cellulose, carbopol, carboxymethyl cellulose sodium, sucrose, metharcylic acid copolymers, gelatine, xanthan gum, and guar gum.

In the dosage form of the invention the binder is present in an amount of 0.5 % to 5 % by weight. Preferably the binder is present in an amount of 1 % to 4 % by weight, more preferably in amount of 1.5 % to 2.5 % by weight, even more preferably in an amount of 2% by weight.

In a further preferred the binder is Povidone K30.

In one embodiment the disintegrant or swelling agent is selected from the group consisting of starches, cellulose, crosslinked polyvinylpyrrolidone (PVP), sodium starch glycolate, alginate, polacrilins, cross carmellose sodium, low substituted hydroxypropylcellulose (HPC), and calcium carboxymethyl cellulose.

In a further preferred embodiment the disintegrant or swelling agent is present in an amount of 5 % to 10 % by weight. Preferably the disintegrant or swelling agent is present in an amount of 5 % to 8 % by weight, more preferably in an amount of 6 % to 7% by weight.

In another preferred embodiment the disintegrant or swelling agent is sodium starch glycolate.

In a further preferred embodiment the sodium starch glycolate is present in an amount of 5 % to 10 % by weight. Preferably the disintegrant or swelling agent is present in an amount of 5 % to 8 % by weight, more preferably in an amount of 6 % to 7% by weight, even more preferably in an amount of 7% by weight.

In one embodiment of the coated solid oral dosage form according to the present invention the additives comprise a glidant, a lubricant, and at least one filler or diluent.

In another embodiment of the coated solid oral dosage form according to the present invention the additives comprise a glidant.

In a preferred embodiment the glidant is selected from the group consisting of colloidal silica, magnesium trisilicate, powdered cellulose, starch, talc, tribasic calcium phosphate, stearic acid, sodium stearyl fumarate, calcium stearate, and sodium lauryl sulfate.

In another preferred embodiment the glidant is colloidal silica, such as Aerosil 200.

In a further preferred embodiment the colloidal silica, such as Aerosil 200 is present in an amount of 0.1 % to 5 % by weight. Preferably colloidal silica, such as Aerosil 200 is present in an amount of 0.1 % to 3 % by weight, more preferably in an amount of 0.1 % to 1 % by weight, even more preferably 0.1 % to 0.5 % by weight.

In one further embodiment of the coated solid oral dosage form according to the present invention the additives comprise a lubricant.

In a preferred embodiment the lubricant is selected from the group consisting of Mg, Al, Ca stearate, PEG 4000-8000, talc, stearic acid, sodium stearyl fumarate, sodium lauryl sulfate, leucin, and hydrogenated vegetable oil.

In another preferred embodiment the lubricant is magnesium stearate.

In a further preferred embodiment the magnesium stearate is present in an amount of 0.1 % to 5 % by weight. Preferably magnesium stearate is present in an amount of 0.1 % to 3 % by weight, more preferably in an amount of 0.1 % to 1 % by weight, even more preferably in an amount of 0.1 % to 0.5 % by weight.

In another embodiment of the coated solid oral dosage form according to the present invention the additives comprise at least one filler or diluent.

In a preferred embodiment the at least one filler or diluent is selected from the group consisting of confectioner's sugar, compressible sugar, dextrates, dextrin, dextrose, lactose, lactose monohydrate, mannitol, microcellulose, powdered cellulose, sorbitol, sucrose and talc.

In another preferred embodiment the at least one filler or diluent is lactose monohydrate and/or microcrystalline cellulose. Preferably the at least one filler or diluent is lactose monohydrate and microcrystalline cellulose.

In a further preferred embodiment the lactose monohydrate is present in an amount of 5 % to 15 % by weight. Preferably lactose monohydrate is present in an amount of 5 % to 10 % by weight, more preferably in an amount of 5 % to 8 % by weight, even more preferably in an amount of 6 % to 7 % by weight.

In another preferred embodiment the microcrystalline cellulose is present in an amount of 3 % to 10 % by weight. Preferably the microcrystalline cellulose is present in an amount of 4 % to 8 % by weight, more preferably in an amount of 5 % to 6 % by weight.

In one embodiment of the coated solid oral dosage form according to the present invention the coating material is selected from the group consisting of hydroxypropylmethyl cellulose, methacrylic acid copolymers, thyl, crystalline cellulose, and povidone, or wherein said coating material is a coating system, comprising a polymer, a plasticizer and, if desired, a pigment in a dry concentrate. Preferably the coating material is a coating system such as Opadry®.

In a preferred embodiment the coating material is Opadry.

In another preferred embodiment the coating system, such as Opadry® is present in an amount of 1 % to 5 % by weight. Preferably the coating system, such as Opadry® is present in an amount of 1% to 4 % by weight, more preferably in an amount of 1% to 2 % by weight.

In a further preferred embodiment of the coated solid oral dosage form according to the present invention the coating material or coating system further comprises a foam control.

In another preferred embodiment the foam control is simethicone emulsion 30%.

In a further preferred embodiment the simethicone emulsion 30% is present in an amount of 0.01 % to 1 % by weight. Preferably the simethicone emulsion 30% present in an amount of 0.01 to 0.05 % by weight, more preferably between 0.015 to 0.025 by weight, even more preferably 0.02 % by weight.

In another preferred embodiment of the coated solid oral dosage form according to the present invention the coating material further comprises a plastisizer.

In a preferred embodiment the plastisizer is selected from the group consisting of polyethylene glycol, propylene glycol, triethyl citrate, triacetin, stearic acid, dibutylphthalate, diethyl phthalate, and dibutyl sebecate.

In another preferred embodiment of the coated solid oral dosage form according to the present invention the coating material further comprises a opicifier.

In a preferred embodiment the opicifier is titanium oxide.

In another preferred embodiment of the coated solid oral dosage form according to the present invention the coating material further comprises a anti-caking agent.

In a preferred embodiment the anti-caking agent is talc or colloidal silicon dioxide.

In one embodiment of the coated solid oral dosage form according to the present invention the coated solid oral dosage form has a Cₘₐₓ of about 3.3 µg/ml when administered as an unit dose of 1000 mg in a single dose human bioavailability study under fed conditions.

In another embodiment of the coated solid oral dosage form according to the present invention the coated solid oral dosage form has an AUC₀₋ₜ of about 18.6 µg.h/ml when administered as an unit dose of 1000 mg in a single dose human bioavailability study under fed conditions.

In one further embodiment of the coated solid oral dosage form according to the present invention the coated solid oral dosage form has an AUC_{0-∞} of about 19.8 µg.h/ml when administered as an unit dose of 1000 mg in a single dose human bioavailability study under fed conditions.

In another embodiment of the coated solid oral dosage form according to the present invention the coated solid oral dosage form has a Cₘₐₓ of about 3.9 µg/ml when administered as an unit dose of 1000 mg in a single dose human bioavailability study under fasting conditions.

In a further embodiment of the coated solid oral dosage form according to the present invention the coated solid oral dosage form has an AUC₀₋ₜ of about 16.4 µg.h/ml when administered as an unit dose of 1000 mg in a single dose human bioavailability study under fasting conditions.

In another embodiment of the coated solid oral dosage form according to the present invention the coated solid oral dosage form has an AUC_{0-∞} of about 18.4 µg.h/ml when administered as an unit dose of 1000 mg in a single dose human bioavailability study under fasting conditions.

In another aspect the present invention relates to the use of the coated solid oral dosage form according to the present invention in the treatment of a disease selected from acute sinusitis, lower respiratory tract infections, acute exacerbations of chronic bronchitis (AECB), urinary tract infections, acute uncomplicated cystitis in females, chronic bacterial prostatitis, complicated intraabdominal infections, skin and skin structure infections, bone and joint infections, infectious diarrhea, typhoid fever, uncomplicated cervical and urethral gonorrhea, nosocominal pneumonia, and empirical therapy for febrile neutropenic patients.

### EXAMPLES

### Example 1: Dissolution profile

Comparison of the dissolution profile between the coated solid oral dosage form of the present invention (Quinox® XR 1g) and the reference Cipro® XR 1g tablet, an extended release tablet of Ciprofloxacin provided by the company Bayer. The test was performed according to the USP monograph of Ciprofloxacin tablet i.e. 0.01 HCl, 900ml, apparatus 2 (Paddle) 50 rpm.

| Time (min) | Cipro® XR 1 g tablet | Formulation of the present invention 1000 mg Quinox® XR 1g |
|---|---|---|
| 0 | 0 | 0 |
| 5 | 16.6 | 7.8 |
| 10 | 32.9 | 31.8 |
| 15 | 43.2 | 53.2 |
| 20 | 49.8 | 70.9 |
| 25 | 53.3 | 84.1 |
| 30 | 56.5 | 92.6 |
| 45 | 70.5 | 98.3 |
| 60 | 82.8 | 98.8 |
| 90 | 96.9 | 98.7 |
| 120 | 101 | 98.8 |
| 150 | 101.9 | 98.8 |
| 180 | 102 | 98.8 |

### Example 2: in vivo behaviour under fed and fasting conditions

The *in vivo* behavior of the coated solid oral dosage form of the present invention (Quinox® XR 1g tablets, test product, (Batch No: SC53)) was compared to the *in vivo* behavior of the reference product Cipro® XR 1 g (Batch No: 25P0243) in a two-way single-dose crossover study.

The study was performed under both fed and fasting conditions using 28 healthy volunteers for each study, whereas under fed conditions the effect of food on the formulated tablets was checked after ingestion of a high fat meal.

The assessment of the two products (test and reference product) was designed in a way leading to an open, crossover, randomized, two treatments, two periods, single dose study in which the test product (Quinox® XR 1g tablets) was compared with the reference product (Cipro® XR 1g tablets) in 28 healthy male volunteers. These volunteers were clinically examined and in addition they gave their informed consent.

This kind of study means that the 28 volunteers have taken both the reference (Cipro® XR 1g tablets) and the test product (Quinox ®XR 1g tablets). On phase one (first week) 14 have taken the reference and 14 have taken the test product, whereas in phase two, the 14 who have taken the reference product were given the test product and the 14 who have taken the test product were given the reference product. In this way there were 28 volunteers who have taken both the test and reference product, further, there were no withdrawals and all 28 volunteers completed the study. The collected plasma samples were analyzed to determine the drug concentration in plasma all over the sampling period and statistical evaluation was done to determine that the two products were bioequivalent or not.

Volunteers were hospitalized the night before the start of the study, they received a standard supper at 8.30 p.m., then they fasted overnight for at least ten hours, and they were given a 1g oral dose of either test or reference drug in accordance with a randomization code. The last step was performed by previously labeled and packed of either the test or the reference product, wherein only one tablet was inserted in a plastic pack. A blood sample was withdrawn through a cannula placed into a suitable forearm or hand vein, before subjects having drug samples, then after post - dosing blood samples were drawn at the following, 0.5, 0.75, 1.0, 1.25, 1.50, 1.75, 2.0, 2.5, 3.0, 3.5, 4.0, 4.5, 5.0, 6.0, 8.0, 10.0, 12.0, 14.0 and 24.0 hours post drug administration.

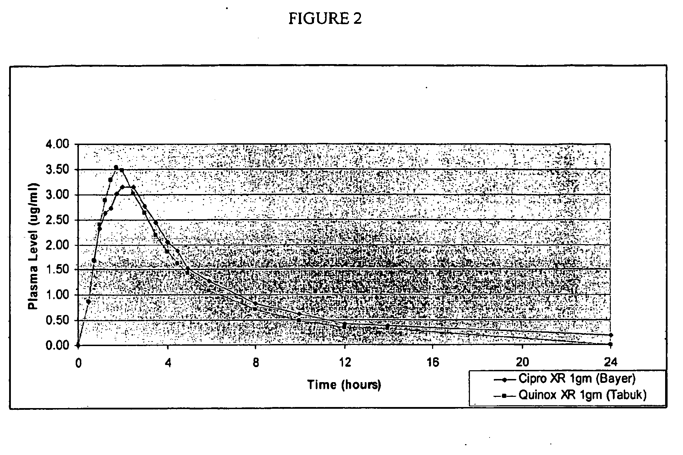

The results showed that the bioavailability (see Figure 2 and tables) of both products Cipro^{®} XR 1 g and the coated solid oral dosage form of the present invention (Quinox® XR 1g) were similar. This was quite surprising since Cipro® XR 1g is an extended release composition using a bilayer matrix, while the coated solid oral dosage form of the present invention releases the drug much faster using a single layer.

In the following the pharmacokinetic parameters of both studies are shown.
A- Under fed conditions:

| **Pharmacokinetic Parameter** | **Cipro® XR 1 g** | **Formulation of the present invention 1000 mg** (Quinox® XR 1g) |
|---|---|---|
| Cmax (µg/ml) | 2.90 | 3.36 |
| Tₘₐₓ (hr) | 2.89 | 2.43 |
| AUC₀₋ₜ (µg.hr/ml) | 19.10 | 18.63 |
| AUC_{0-∞} (µg.hr/ml) | 20.17 | 19.89 |

Data for 90 % Confidence intervals are presented in the following:

| **Pharmacokinetic Parameter** | **90 % Confidence Interval** |
|---|---|
| AUC_{0.t} (µg.hr/ml) | 93.68 - 100.83 %( Limit : 80.0 - 125.0%) |
| AUC_{0.∞} (µg.hr/ml) | 94.92 - 101.67 %( Limit : 80.0 - 125.0% ) |
| Cₘₐₓ (µg/ml) | 106.15 -122.94 %( Limit : 80.0 -125.0% ) |

B- Under fasting conditions:

| **Pharmacokinetic Parameter** | **Cipro® XR 1g** | **Formulation of the present invention 1000 mg** (Quinox® XR 1g) 1000 |
|---|---|---|
| Cₘₐₓ (µg/ml) | 3.69 | 3.96 |
| Tₘₐₓ (hr) | 1.96 | 1.75 |
| AVC_{0.t} (µg.hr/ml) | 17.52 | 16.46 |
| AVC_{0.∞} (µg.hr/ml) | 19.63 | 18.43 |
| T_{1/2} (hr) | 4.13 | 4.28 |

Data for 90 % Confidence intervals are presented in the following tablets:

| **Pharmacokinetic Parameter** | **90 % Confidence Interval** |
|---|---|
| AUC ₀₋ₜ (µg.hr/ml) | 80.24-107.7% ( Limit : 80.0-125.0%) |
| AUC_{0-∞} (µg.hr/ml) | 81.74-113.8% ( Limit : 80.0 - 125.0%) |
| Cₘₐₓ (µg/ml) | 81.42-113.65% ( Limit : 80.0 - 125.0%) |

In summary, the values of the 90% confidence level for the primary pharmacokinetic parameters of the two products of ciprofloxacin are within acceptable limits. This was confirmed by the fact that the sum of the probability at the upper and lower percentile was not less than 0.9, which is within the acceptable range for the active ingredient. Further, no significant difference was established between the peak plasma levels (Cₘₐₓ), produced by both brands for ciprofloxacin. The obtained data were within the acceptance criteria for bioequivalence as defined by United States Food and Drug Administration and by the World Health Organization.

This has been concluded on the basis that the two-one sided probability which was estimated was again within the 0.9 accepted level.

The rate of decline (see figure 2) of Cipro® XR 1g and Quinox® XR 1g plasma concentration, as indicated by the terminal segments of the plasma profile was similar for both products.

It was concluded, on the basis of the results obtained from this study, that the test drug product Quinox® XR 1g tablets was bioequivalent with the reference brand Cipro® XR 1g tablets. Thus, Quinox® XR and Cipro® XR tablets, each containing 1g ciprofloxacin, are bioequivalent with regard to C ₘₐₓ, AUC_{0→t} and AUC_{0→∞}. Furthermore, there were no safety concerns.

### Example 3: Different compositions of the inventive coated oral dosage form

| Ingredient | Quantity ( mg ) | | | | | | | |
|---|---|---|---|---|---|---|---|---|
| | Example 1 | Example 2 | Example 3 | Example 4 | Example 5 | Example 6 | Example 7 | Example 8 |
| Ciprofloxacin Hydrochloride | 669.3 | 669.3 | 669.3 | 669.3 | 669.3 | 669.3 | 669.3 | 669.3 |
| Ciprofloxacin base | 425.2 | 425.2 | 425.2 | 425.2 | 425.2 | 425.2 | 425.2 | 425.2 |
| Lactose Monohydrate | 64.4 | 64.4 | 64.4 | 92.4 | 85.4 | 92.4 | 92.4 | 92.4 |
| Povidone K30 | 84 | 84 | 70 | 42 | 49 | 42 | 28 | 28 |
| Colloidal Silicone Dioxide | 4.2 | 4.2 | 4.2 | 4.2 | 4.2 | 4.2 | 4.2 | 4.2 |
| Magnesium Stearate | 7 | 7 | 7 | 14 | 10.5 | 14 | 14 | 7 |
| Microcrystalline Cellulose | 75.7 | 75.7 | 61.9 | 61.9 | 61.9 | 54.9 | 68.9 | 75.9 |
| Sodium Starch Glycolate | 70 | 70 | 98 | 98 | 98 | 98 | 98 | 98 |
| Opadry | 27.7 | 27.7 | 27.7 | 27.7 | 27.7 | 27.7 | 27.7 | 27.7 |
| Simethicone Emulsion 30% | 0.3 | 0.3 | 0.3 | 0.3 | 0.3 | 0.3 | 0.3 | 0.3 |

## Claims

1. A coated solid mono-layer oral dosage form comprising
a) an active agent containing an effective amount of Ciprofloxacin hydrochloride and Ciprofloxacin base, wherein the active agent is present in an amount of 60% to 80% by weight based on the total weight of the dosage form;
b) one or more further pharmaceutically acceptable excipients suitable for the preparation of the dosage form comprising a binder and a disintegrant or swelling agent wherein the binder is selected from the group consisting of starches, microcrystalline cellulose, Povidone K30, hydroxypropyl cellulose, hydroxyl ethyl cellulose, hydroxypropylmethyl cellulose, carbopol, carboxymethyl cellulose sodium, sucrose, methacrylic acid copolymers, gelatine, xanthan gum and guar gum and is contained in an amount of 0.5% to 5% by weight based on the total weight of the dosage form; and
c) coating materials.

2. The coated solid oral dosage form according to claim 1, wherein the binder is present in an amount of 1% to 4% by weight.

3. The coated solid oral dosage form according to claim 2, wherein the binder is present in an amount of 1.5% to 2.5% by weight.

4. The coated solid oral dosage form according to claim 3, wherein the binder is present in an amount of 2% by weight.

5. The coated solid oral dosage form according to any of claims 1 to 4, wherein said binder is Povidone K30.

6. The coated solid oral dosage form according to any of claims 1 to 5, wherein said active agent is present in an unit dose of 500 mg or 1000 mg.

7. The coated solid oral dosage form according to any of claims 1 to 6, wherein said Ciprofloxacin hydrochloride is present in an amount of 40 % to 50 % by weight based on the total weight of coated solid oral dosage forms.

8. The coated solid oral dosage form according to any of claims 1 to 7, wherein said Ciprofloxacin base is present in an amount of 25 % to 35 % by weight based on the total weight of coated solid oral dosage forms.

9. The coated solid oral dosage form according to any of claims 1 to 8, wherein said disintegrant or swelling agent is selected from the group consisting of starches, cellulose, crosslinked polyvinylpyrrolidone (PVP), sodium starch glycolate, alginate, polacrilins, cross carmellose sodium, low substituted hydroxyl propyl cellulose (HPC), and calcium carboxymethyl cellulose.

10. The coated solid oral dosage form according to claim 9, wherein said disintegrant or swelling agent is present in an amount of 5 % to 10 % by weight based on the total weight of coated solid oral dosage forms.

11. The coated solid oral dosage form according to claim 10, wherein said disintegrant or swelling agent is sodium starch glycolate.

12. The coated solid oral dosage form according to any of claims 1 to 11, wherein said excipients additionally comprise a glidant, a lubricant, and at least one filler or diluent.

13. The coated solid oral dosage form according to any of claims 1 to 12, wherein said coating material is selected from the group consisting of hydroxypropylmethyl cellulose, methacrylic acid copolymers, thyl, crystalline cellulose, and povidone, or wherein said coating material is a coating system, comprising a polymer, a plasticizer and optionally a pigment.

## Patentansprüche

1. Beschichtete feste einschichtige orale Darreichungsform, umfassend:
a) einen Wirkstoff, der eine wirksame Menge Ciprofloxacin-Hydrochlorid und Ciprofloxacin-Base enthält, wobei der Wirkstoff in einer Menge von 60 bis 80 Gew.-%, bezogen auf das Gesamtgewicht der Darreichungsform, vorliegt;
b) einen oder mehrere weitere pharmazeutisch annehmbare Hilfsstoffe, die zur Herstellung der Darreichungsform geeignet sind, umfassend ein Bindemittel und ein Desintegrationsmittel oder Quellmittel, wobei das Bindemittel ausgewählt ist aus der Gruppe, bestehend aus Stärken, mikrokristalliner Cellulose, Povidon K30, Hydroxypropylcellulose, Hydroxyethylcellulose, Hydroxypropylmethylcellulose, Carbopol, Carboxymethylcellulose-Natrium, Saccharose, Methacrylsäure-Copolymeren, Gelatine, Xanthangummi und Guargummi, und in einer Menge von 0,5 bis 5 Gew.-%, bezogen auf das Gesamtgewicht der Darreichungsform, enthalten ist; und
c) Beschichtungsmaterialien.

2. Beschichtete feste orale Darreichungsform nach Anspruch 1, wobei das Bindemittel in einer Menge von 1 bis 4 Gew.-% vorliegt.

3. Beschichtete feste orale Darreichungsform nach Anspruch 2, wobei das Bindemittel in einer Menge von 1,5 bis 2,5 Gew.-% vorliegt.

4. Beschichtete feste orale Darreichungsform nach Anspruch 3, wobei das Bindemittel in einer Menge von 2 Gew.-% vorliegt.

5. Beschichtete feste orale Darreichungsform nach einem der Ansprüche 1 bis 4, wobei das Bindemittel Povidon K30 ist.

6. Beschichtete feste orale Darreichungsform nach einem der Ansprüche 1 bis 5, wobei der Wirkstoff in einer Einheitsdosis von 500 mg oder 1.000 mg vorliegt.

7. Beschichtete feste orale Darreichungsform nach einem der Ansprüche 1 bis 6, wobei das Ciprofloxacin-Hydrochlorid in einer Menge von 40 bis 50 Gew.-%, bezogen auf das Gesamtgewicht der beschichteten festen oralen Darreichungsformen, vorliegt.

8. Beschichtete feste orale Darreichungsform nach einem der Ansprüche 1 bis 7, wobei die Ciprofloxacin-Base in einer Menge von 25 bis 35 Gew.-%, bezogen auf das Gesamtgewicht der beschichteten festen oralen Darreichungsformen, vorliegt.

9. Beschichtete feste orale Darreichungsform nach einem der Ansprüche 1 bis 8, wobei das Desintegrationsmittel oder Quellmittel ausgewählt ist aus der Gruppe, bestehend aus Stärke, Cellulose, vernetztem Polyvinylpyrrolidon (PVP), Natriumstärkeglykolat, Alginat, Polyacrilinen, Crosscarmellose-Natrium, niedrigsubstituierter Hydroxypropylcellulose (HPC) und Calciumcarboxymethylcellulose.

10. Beschichtete feste orale Darreichungsform nach Anspruch 9, wobei das Desintegrationsmittel oder Quellmittel in einer Menge von 5 bis 10 Gew.-%, bezogen auf das Gesamtgewicht der beschichteten festen oralen Darreichungsformen, vorliegt.

11. Beschichtete feste orale Darreichungsform nach Anspruch 10, wobei das Desintegrationsmittel oder Quellmittel Natriumstärkeglykolat ist.

12. Beschichtete feste orale Darreichungsform nach einem der Ansprüche 1 bis 11, wobei die Hilfsstoffe zusätzlich ein Gleitmittel, ein Schmiermittel und wenigstens einen Füllstoff oder ein Verdünnungsmittel umfassen.

13. Beschichtete feste orale Darreichungsform nach einem der Ansprüche 1 bis 12, wobei das Beschichtungsmaterial ausgewählt ist aus der Gruppe, bestehend aus Hydroxypropylmethylcellulose, Methacrylsäure-Copolymeren, Thyl, kristalliner Cellulose und Povidon, oder wobei das Beschichtungsmaterial ein Beschichtungssystem ist, das ein Polymer, einen Weichmacher und ggf. ein Pigment umfasst.

## Revendications

1. Forme galénique monocouche solide enduite pour une administration par voie orale comprenant :
a) un agent actif contenant une quantité efficace du chlorhydrate de Ciprofloxacine et d'une base de Ciprofloxacine, l'agent actif étant présent en une quantité allant de 60 % à 80 % en poids en se basant sur le poids total de la forme galénique ;
b) un ou plusieurs autres excipients pharmaceutiquement acceptables appropriés pour la préparation de la forme galénique comprenant un liant et un délitant ou un agent gonflant, le liant étant choisi dans le groupe comprenant des amidons, de la cellulose microcristalline, de la Povidone K30, de l'hydroxypropyl-cellulose, de l'hydroxyéthyl-cellulose, de l'hydroxypropylméthyl-cellulose, du carbopol, de la carboxyméthyl-cellulose sodique, du saccharose, des copolymères de l'acide méthacrylique, de la gélatine, de la gomme xanthane et de la gomme guar et étant présent en une quantité allant de 0,5 % à 5 % en poids en se basant sur le poids total de la forme galénique ; et
c) des substances de revêtement.

2. Forme galénique solide enduite pour une administration par voie orale selon la revendication 1, dans laquelle le liant est présent en une quantité allant de 1 % à 4 % en poids.

3. Forme galénique solide enduite pour une administration par voie orale selon la revendication 2, dans laquelle le liant est présent en une quantité allant de 1,5 % à 2,5 % en poids.

4. Forme galénique solide enduite pour une administration par voie orale selon la revendication 3, dans laquelle le liant est présent en une quantité de 2 % en poids.

5. Forme galénique solide enduite pour une administration par voie orale selon l'une quelconque des revendications 1 à 4, dans laquelle ledit liant est de la Povidone K30.

6. Forme galénique solide enduite pour une administration par voie orale selon l'une quelconque des revendications 1 à 5, dans laquelle ledit agent actif est présent en une dose unitaire de 500 mg ou 1 000 mg.

7. Forme galénique solide enduite pour une administration par voie orale selon l'une quelconque des revendications 1 à 6, dans laquelle ledit chlorhydrate de Ciprofloxacine est présent en une quantité allant de 40 % à 50 % en poids en se basant sur le poids total des formes galéniques solides enduites pour une administration par voie orale.

8. Forme galénique solide enduite pour une administration par voie orale selon l'une quelconque des revendications 1 à 7, dans laquelle ladite base de Ciprofloxacine est présente en une quantité allant de 25 % à 35 % en poids en se basant sur le poids total des formes galéniques solides enduites pour une administration par voie orale.

9. Forme galénique solide enduite pour une administration par voie orale selon l'une quelconque des revendications 1 à 8, dans laquelle ledit délitant ou ledit agent gonflant est choisi dans le groupe comprenant des amidons, de la cellulose, de la polyvinylpyrrolidone réticulée (PVP), du glycolate d'amidon sodique, un alginate, des polacrilines, de la carmellose sodique réticulée, de l'hydroxypropylcellulose (HPC) faiblement substituée, et de la carboxyméthyl-cellulose calcique.

10. Forme galénique solide enduite pour une administration par voie orale selon la revendication 9, dans laquelle ledit délitant ou ledit agent gonflant est présent en une quantité allant de 5 % à 10 % en poids en se basant sur le poids total des formes galéniques solides enduites pour une administration par voie orale.

11. Forme galénique solide enduite pour une administration par voie orale selon la revendication 10, dans laquelle ledit délitant ou ledit agent gonflant est du glycolate d'amidon sodique.

12. Forme galénique solide enduite pour une administration par voie orale selon l'une quelconque des revendications 1 à 11, dans laquelle lesdits excipients comprennent de plus un agent de glissement, un lubrifiant, et au moins une charge ou un diluant.

13. Forme galénique solide enduite pour une administration par voie orale selon l'une quelconque des revendications 1 à 12, dans laquelle ladite substance de revêtement est choisie dans le groupe comprenant de l'hydroxypropylméthyl-cellulose, des copolymères de l'acide méthacrylique, du thyl, de la cellulose cristalline, et de la povidone, ou dans laquelle ladite substance de revêtement est un système de revêtement, comprenant un polymère, un plastifiant et éventuellement un pigment.
